Europäisches Patentamt

European Patent Office

Office européen des brevets

(11) Publication number: **0 160 994**
**A2**

## EUROPEAN PATENT APPLICATION

(21) Application number: **85105685.3**

(22) Date of filing: **09.05.85**

(51) Int. Cl.⁴: **A 61 B 5/02**

(30) Priority: **10.05.84 US 608955**

(43) Date of publication of application:
**13.11.85 Bulletin 85/46**

(84) Designated Contracting States:
**AT BE CH DE FR GB IT LI LU NL SE**

(71) Applicant: **Warner, Sylvia**
**3010 Matis Street**
**St. Laurent Quebec H4R 1A3(CA)**

(72) Inventor: **Warner, Glenfield**
**3010 Matis Street**
**Saint-Lauren Quebec H4R 1A3(CA)**

(74) Representative: **Casalonga, Axel et al,**
**BUREAU D.A. CASALONGA OFFICE JOSSE & PETIT**
**Baaderstrasse 12-14**
**D-8000 München 5(DE)**

(71) Applicant: **Sankar, Priyamvada**
**8000 Nantes Street**
**Brossard Quebec J4Y 1Z1(CA)**

(54) **Heart-related parameters monitoring apparatus.**

(57) A non-invasive method, and an apparatus, for determining heart-related parameters in patients. The method and apparatus determine pulse pressure, time constant of the arterial system, systolic and diastolic pressure, peripheral resistance, cardiac output and mean arterial blood pressure.

PLETHYSMO-GRAPH AMPLIFIER — MICRO-COMPUTER — A/D CONVERTER — DISPLAY — PHOTO-PLETHYMO-GRAPH

Fig. 1

EP 0 160 994 A2

The invention relates to a non-invasive method of measuring arterial blood pressure and cardiac output. The invention also relates to an apparatus for carrying out the method.

Non-invasive methods and apparatus for measuring arterial blood pressure and cardiac output are known in the art. One such method and apparatus is illustrated in U.S. Patent 4,030,485 Warner, issued June 21,1977. A second such method and apparatus is taught in U.S. Patent 4,418,700, Warner, issued December 6,1983. The present invention constitutes an improvement and refinement of the method and apparatus as taught in the latter patent.

The invention relates to a non-invasive method, and an apparatus, for determining heart-related parameters in patients. The Method and apparatus determine pulse pressure, time constant of the arterial system, systolic and diastolic pressure, peripheral resistance, and cardiac output and mean arterial blood pressure.

The invention will be better understood by an examination of the following description together with the accompanying drawings in which :

        Figure 1 is a block diagram of the apparatus
              for carrying out the inventive method;

        Figure 2 is a typical sensor output of the system
              as illustrated in Figure 1;

        Figure 3 illustrates arterial blood pressure pulses;

        Figure 4 illustrates a blood volume pulse;

        Figure 5 illustrates a blood volum pulse and a
              blood pressure pulse to illustrate the
              ratio g; and

        Figure 6 is a simplified flowchart for a computer
              program for performing calculations in
              accordance with the invention.

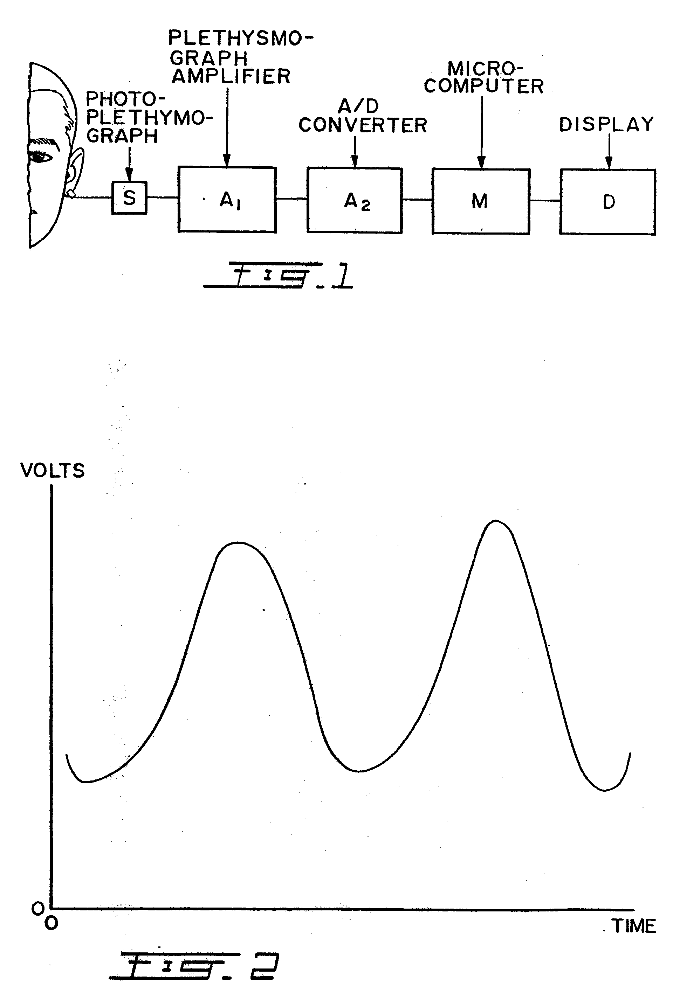

As seen in Figure 1, an apparatus in accordance with the invention comprises a volume sensor such as a photo-electric plethysmograph S, an amplifier $A_1$, an analog to digital converter $A_2$, a microcomputer M and a display device D. The plethysmograph sensor S is attached to, for example, the earlobe of a subject.

0160994

The sensor could also be attached to other suitable parts of the body such as the forehead, fingertips or toes.

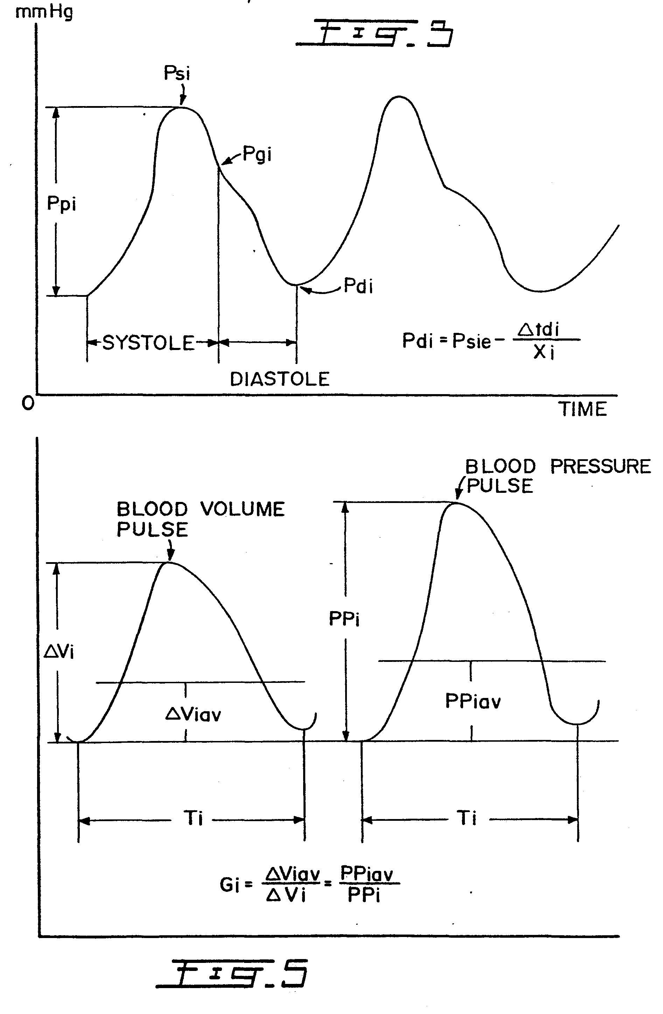

As is known, the plethysmograph detects changes in blood volume of the region to which it is attached. A typical sensor output signal is shown in Figure 2. As seen in Figure 2, the output signal has a pulsating component and a DC component.

The output of the sensor is applied to the plethysmograph amplifier $A_1$ where it si amplified and filtered and the DC component is discarded. The output of $A_1$ has a DC component, but this is not directly related to the sensor DC component.

The output of $A_1$ is fed to the analog to digital (A/D) converter $A_2$ which digitizes the signal. In a preferred embodiment, the sampling rate is 100 per second.

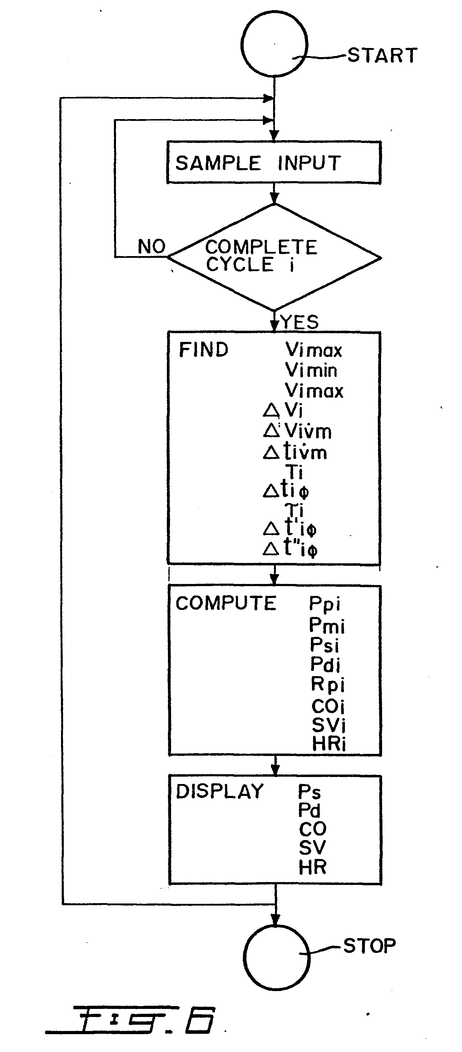

Microcomputer M accepts signals from $A_2$ and processes them according to the instructions it contains. These instructions are schematically represented in the simplified flowchart of Figure 6.

The computer quantities are than displayed on a CRT monitor D or other suitable display means.

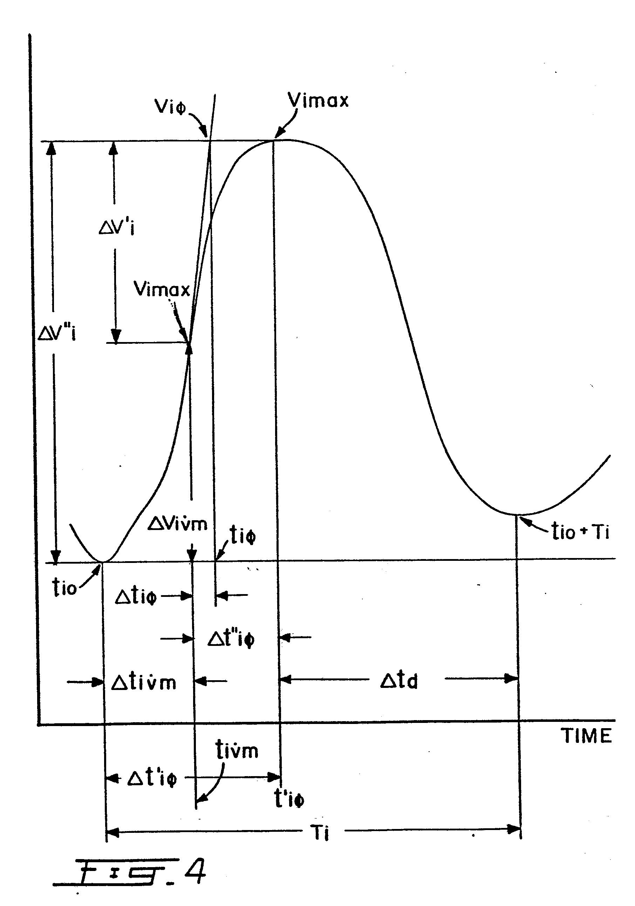

Arterial blood pressure pulses are shown in Figure 3. The shape of these curves vary according to the site where they are measured. The highest pressure reached during a cycle i is called the arterial systolic blood pressure, $P_{si}$. The lowest pressure reached during the same cycle is called the arterial diastolic blood pressure, $P_{di}$. The pressure rise from $P_{di}$ to $P_{si}$ in the same cycle is the pulse pressure , $P_{pi}$.

By definition $\quad\quad P_{si} - P_{di} = P_{pi}$ $\quad\quad\quad$ (1)

A plethysmograhic pulse is shown in Figure 4. The minimum value at the beginning of the pulse is $V_{imin}$. The maximum value of the pulse is $V_{imax}$. As the pulse volume rises from $V_{imin}$ to $V_{imax}$, the time rate of volume change reaches a maximum $\dot{V}_{imax}$ at time $t_{i\dot{V}m}$. The pulse volume at time $t_{i\dot{V}m}$ is $V_{i\dot{V}m}$.

$$\text{let} \quad \frac{V_{i\dot{V}m} - V_{imin}}{V_{imax} - V_{imin}} = \frac{\Delta V_{i\dot{V}m}}{\Delta V_i} = R_{i1} \quad (2)$$

also let

$$\frac{t_{i\dot{V}m} - t_{io}}{t'_{i\phi} - t_{io}} = \frac{\Delta t_{i\dot{V}m}}{\Delta t'_{i\phi}} = R_{i2}$$ <span>0160994</span>

where $\Delta V_{i\dot{V}m}$ = volume change at time $t_{i\dot{V}m}$ during cycle i corresponding to maximum rate of volume change , $\dot{V}_{imax}$

$\Delta V_i$ = maximum volume change during cycle i

$\Delta t_{i\dot{V}m}$ = time interval from start of cycle i to time of maximum rate of volume change $\dot{V}_{imax}$

$\Delta t'_{i\phi}$ = time interval from start of cycle i to time of maximum volume $V_{imax}$

$$\Delta V_i = \Delta V_{i\dot{V}m} + \frac{\dot{V}_{imax}(\Delta t''_{i\phi})}{2} \quad ; \text{ or } = \Delta V''_i$$

In addition to finding the value of $V_{i\dot{V}m}$ corresponding to $V_{imax}$, see U.S. Patent 4,418,700, Warner , values of $V_{i\dot{V}m}$ are also found corresponding to $\dot{V}_{imax}-1$ , $\dot{V}_{imax}-2, \ldots \dot{V}_{imax}-k$ , where K is a function of $V_{imax}$.

The average value of $V_{i\dot{V}m}$ is

$$\overline{V}_{i\dot{V}m} = \frac{\sum_1^{n0} V_{i\dot{V}_1}mn1 + \sum_1^{n1} V_{i\dot{V}_2}mn2 + \ldots + \sum_1^{nk} V_{i\dot{V}_k}mnk}{n0 + n1 + \ldots + nk}$$

where $n0$ = number of values of $V_{i\dot{V}_1 m}$ corresponding to $\dot{V}_{imax}$

$n1$ = number of values of $V_{i\dot{V}_2 m}$ corresponding to $\dot{V}_{imax}-1$

$\ldots$ = $\ldots\ldots\ldots\ldots\ldots\ldots\ldots\ldots\ldots\ldots\ldots\ldots\ldots\ldots\ldots\ldots\ldots\ldots\ldots$
$\ldots\ldots\ldots\ldots\ldots\ldots$

$nk$ = number of values of $V_{i\dot{V}_k m}$ corresponding to $\dot{V}_{imax}-k$

$k = \dfrac{\dot{V}_{imax}}{m}$ (integral values only) $+ \ell$

$m$ = constant ... a preferred value of m = 20

$\ell$ = constant ... a preferred value of $\ell$ = 1

let $R_i = \dfrac{R_{i1} + R_{i2}}{2}$

then $PP_i = K_p \ln(1-R_i)$ \qquad\qquad (4)

$$\text{or} \quad PP_i \;=\; K_{pp} \left( \frac{R_i + r_1}{r_1 + r_2 - R_i} \right)$$

where $K_p$ = constant determined by a first calibration

$K_{pp}$ = constant determined by a first calibration

$r_1$ = constant... preferably equal to 0

$r_2$ = constant ... preferably equal to 0.

In equations 4 and 4a, $R_i$ can be replaced by $R_{i1}$ or $R_{i2}$. No other calibration is required with different subjects. However, if desired, $K_{pp}$ and $K_p$ can be determined for each subject.

The mean blood pressure $P_{mi}$ during a cycle i is given by

$$P_{mmi} \;=\; K_4 \left[ \frac{\Delta V_i}{\Delta \dot{V}_{imax}} \right]^{-b_3} \tag{5}$$

$$P_{mi} \;=\; P_{mmi} + P_0 \tag{6}$$

$b_3$ = exponent ... the preferred value of $b_3$ is equal to 0.5

$K_4$ = constant determined at calibration for each subject. It is only necessary to find this constant once for each subject. The measurements carried out at different times on the same subject do not require separate calibration.

$P_0$ = constant ... preferred 25 mmHg

$$P_{si} \;=\; P_{mi} + (1-g_i) \, P_{pi} \tag{7}$$

where $g_i$ = $\dfrac{\Delta V_{iAV}}{\Delta V_i}$

$$P_{di} \;=\; P_{si} - P_{pi} \tag{8}$$

The variable $g_i$ can take on a constant value $g_0$ whose preferred value is 0.333.

An angle $\emptyset_i$ can be defined as

$$\emptyset_i \;=\; K_\emptyset \, 360 \, \frac{\Delta t_{i\emptyset}}{T_i} \quad \text{degrees} \tag{9}$$

where $\Delta t_{i0} = \Delta \dot{V}_i / \dot{V}_{imax}$

$K_0 = $ constant to be determined at first calibration

then $R_{pi} = K_R \left[ \dfrac{\tau_i}{\omega_i \tan\phi_i} \right]^{a_1}$

$R_{pi} = $ average peripheral resistance during cycle i

$\omega_i = $ circular frequency

$K_R = $ constant

$a_1 = $ exponent ... preferably equal to 1/2

$\tau_i = \dfrac{\Delta t_{di}}{\ln \left( \dfrac{P_{di}-P_o}{P_{si}-P_o} \right)}$

The cardiac output can be found from the pseudo mean pressure $P_{mmi}$ and the peripheral resistance $R_{pi}$.

Cardiac output C.O. equals

$$CO_i = 60 \ \frac{P_{mmi}}{R_{pi}} \quad \text{litres/min.} \qquad (10)$$

Average stroke Volume S.V. is

$$SV_i = \frac{CO_i}{HR_i} \quad \text{litres} \qquad (11)$$

where $HR_i = $ instantaneous heart rate in beats/min. corresponding to cycle i.

Other quantities such as stroke work, arterial uptake , arterial compliance can be calculated from the basic quantities computed.

Although particular embodiments have been illustrated, this was for the purpose of describing, but not limiting, the invention. Various modifications, which will come readily to the mind of one skilled in the art, are within the scope of the invention as defined in the appended claims.

I CLAIM,

1.        Apparatus for determining the magnitude of heart-related parameters in a patient;

comprising:

means for detecting blood volume, and thereby blood volume variation, in said patient, and for providing a signal representative of said blood volume, and thereby said blood volume variation;

said means for detecting being attachable to said patient to thereby detect said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said minimum amplitude and said maximum amplitude, a maximum rate of change of said signal being representative of the maximum rate of increase of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum rate of change  of said signal being representative of the difference in volume between the maximum amount of blood volume and the volume at the time of maximum
rate of change of said blood volume, and a pulse
repetition period;

means for measuring said maximum amplitude,
said minimum amplitude, said maximum rate of change
of said signal, said first difference, said second
difference, said first time interval, and said
second time interval; and

means for calculating the magnitude of
selected ones of said parameters, said means for
calculating being connected to both said means for
detecting and means for measuring;

wherein means for calculating calculates
the magnitude of the selected parameter in accordance with a preferred expression.

2.      An apparatus as defined in claim 1 wherein
said selected parameter is pulse pressure and wherein
the predetermined expression is:

$$P_{pi} = K_p \ln (1-R_i)$$

wherein

$P_{pi}$ = pulse pressure during cycle i

$K_p$ = constant determined by a first
calibration.

3.      An apparatus as defined in claim 1 wherein said selected parameter is pulse pressure and wherein the predetermined expression is:

$$P_{pi} = K_{pp} \left( \frac{R_i + r_1}{1 + r_2 - R_i} \right)$$

wherein

$P_{pi}$ = pulse pressure during cycle i

$K_{pp}$ = constant determined by a first calibration

$r_1$ = constant

$r_2$ = constant.

4.      An apparatus as defined in claim 3 wherein said selected parameter is the mean arterial blood pressure, $P_{mi}$ and wherein the predetermined expression is:

$$P_{mmi} = K_4 \left[ \frac{\Delta V_i}{\Delta V_{imax}} \right]^{-b_3}$$

$$P_{mi} = P_{mmi} + P_0$$

wherein

$K_4$ = constant determined for each subject

$P_{mi}$ = mean arterial blood pressure during cycle i

$b_3$ = constant

$P_0$ = constant

$P_{mmi}$ = pseudo mean arterial blood pressure during cycle i.

5.     An apparatus as defined in claim 4 wherein said selected parameter is systolic pressure ($P_{si}$) and wherein said predetermined expression is:

$$P_{si} = P_{mi} + (1-g_0)P_{pi}$$

wherein

$g_0$ = constant          preferred value = 0.333.

6.     An apparatus as defined in claim 5 wherein said selected parameter is systolic pressure $P_{si}$ and wherein said predetermined expression is:

$$P_{si} = P_{mi}+(1-g_i)P_p$$

wherein:

$$g_i = \frac{\Delta V_{iAV}}{\Delta V_i}$$

$\Delta V_i$ = represented by said first difference

$\Delta V_{iAV}$ = represented by the difference between said minimum amplitude and an amplitude equal to the average value of a pulse in a cycle i.

7.     An apparatus as defined in claim 6 wherein said selected parameter is peripheral resistance ($R_{pi}$) and wherein said predetermined expression is:

$$R_{pi} = K_R \left(\frac{\tau_i}{\omega_i \tan\phi_i}\right)^{a_1}$$

wherein

$K_R$ = constant

$\omega_i$ = circular frequency during cycle i

$\phi_i = K_\phi \, 360 \cdot \dfrac{\Delta t_{i\phi}}{T_i}$

$\Delta t_{i\phi}$ = said second time interval during cycle i

$K_O$ = constant

$T_i$ = said pulse repetition period associated with cycle i

$a_1$ = constant preferred value = 0.5

$$\mathcal{T}_i = \frac{\Delta t_{di}}{\ell n\left(\dfrac{P_{di}-P_O}{P_{si}-P_O}\right)}$$

8.    An apparatus as defined in claim 7 wherein said selected parameter is cardiac output $CO_i$ and wherein said predetermined expression is:

$$CO_i = 60\ \frac{P_{mmi}}{R_{pi}} \quad mL/min$$

wherein

$P_{mmi}$ = pseudo mean arterial pressure.

9.    An apparatus as defined in claim 2 wherein said selected parameter is the mean arterial blood pressure, $P_{mi}$, and wherein the predetermined expression is:

$$P_{mi} = P_{mmi} + P_O$$

$$P_{mmi} = K_4\left[\frac{\Delta V_i}{\Delta V_{imax}}\right]^{-b_3}$$

wherein

$K_4$ = constant determined for each subject

$P_{mi}$ = mean arterial blood pressure during cycle i

$b_3$ = constant

$P_{mmi}$ = pseudo mean arterial blood pressure during cycle i

$P_O$ = constant.

10. An apparatus as defined in claim 9 wherein said selected parameter is systolic pressure ($P_{si}$) and wherein said predetermined expression is:

$$P_{mi} + (1-g_o)P_{pi}$$

wherein

$g_o$ = constant          preferred value = 0.333.

11. An apparatus as defined in claim 10 wherein said selected parameter is systolic pressure, $P_{si}$, and wherein said predetermined expression is:

$$P_{si} = P_{mi} + (1-g_i)P_{pi}$$

wherein

$$g_i = \frac{\Delta V_{iAV}}{\Delta V_i}$$

$\Delta V_i$ = represented by said first difference

$\Delta V_{iAV}$ = represented by the difference between said minimum amplitude and an amplitude equal to the average value of a pulse in a cycle i.

12. An apparatus as defined in claim 11 wherein said selected parameter is peripheral resistance ($R_{pi}$) and wherein said predetermined expression is:

$$R_{pi} = K_R \left( \frac{\gamma_i}{\omega_i \tan\phi_i} \right)^{1/2}$$

wherein

$\omega_i$ = angular frequency of cycle i

$$\phi_i = K_\phi \, 360 \cdot \frac{\Delta t_{i\phi}}{T_i}$$

$\Delta t_{i\phi}$ = said second time interval

$K_\phi$ = constant

$T_i$ = said pulse repetition period

$$\tau_i = \frac{\Delta t_{di}}{\ln\left(\dfrac{P_{di}-P_o}{P_{si}-P_o}\right)}$$

$$P_o = \text{constant.}$$

13.    An apparatus as defined in claim 12 wherein said selected parameter is cardiac output, $CO_i$, and wherein said predetermined expression is:

$$CO_i = 60\,\frac{P_{mmi}}{R_{pi}} \qquad \text{mL/min}$$

wherein

$P_{mmi}$ = pseudo mean arterial pressure.

14.    A method for determining the magnitude of heart-related parameters in a patient;

comprising:

detecting blood volume, and thereby blood volume variation, in said patient and providing a signal representative of said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said maximum amplitude and said minimum amplitude, a maximum rate of change of said signal being representative of the maximum rate of change of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum change of rate of said signal being representative of the difference in volume between the maximum amount of blood volume and the volume at the time of maximum rate of change of said blood volume, and a pulse repetition period;

measuring said maximum amplitude, said minimum amplitude, said maximum rate of change of said signal, said first difference, said second difference, said first time interval, and said second time interval; and

calculating the magnitude of a selected one of said parameters in accordance with a predetermined expression.

15. A method as defined in claim 14 wherein said selected parameter is the mean arterial blood pressure, $P_{mi}$, and wherein the predetermined expression is:

$$P_{mmi} = K_4 \left[ \frac{\Delta V_i}{\Delta V_{imax}} \right]^{-b_3}$$

$$P_{mi} = P_{mmi} + P_0$$

wherein

$K_4$ = constant determined for each subject

$P_{mi}$ = mean arterial blood pressure during cycle i

$b_3$ = constant

$P_0$ = constant

$P_{mmi}$ = pseudo mean arterial blood pressure.

16. A method as defined in claim 15 wherein said selected parameter is systolic pressure ($P_{si}$) and wherein said predetermined expression is:

$$P_{si} = P_{mi} + (1-g_0)P_{pi}$$

wherein

$$g_0 = \text{constant} \qquad \text{preferred value} = 0.333.$$

17. A method as defined in claim 16 wherein said selected parameter is systolic pressure, $P_{si}$, and wherein said predetermined expression is:

$$P_{si} = P_{mi}+(1-g_i)P_{pi}$$

wherein:

$$g_i = \frac{\Delta V_{iAV}}{\Delta V_i}$$

$\Delta V_i$ = represented by said first difference

$\Delta V_{iAV}$ = represented by the difference between said minimum amplitude and an amplitude equal to the average value of a pulse in a cycle i.

18. A method as defined in claim 14 wherein said selected parameter is peripheral resistance ($R_{pi}$) and wherein said predetermined expression is:

$$R_{pi} = K_R \left( \frac{\gamma_i}{\omega_i \tan\phi_i} \right)^{1/2}$$

wherein

$\omega_i$ = angular frequency

$\phi_i = K_\phi \, 360 \cdot \dfrac{\Delta t_{i\phi}}{T_i}$

$\Delta t_{i\phi}$ = said second time interval

$K_\phi$ = a constant

$T_i$ = said pulse repetition period

$$\tau_i \;=\; \frac{\Delta t_{di}}{\ell n \left( \dfrac{P_{di}-P_o}{P_{si}-P_o} \right)}$$

$$P_o \;=\; \text{constant.}$$

19.     A method as defined in claim 14 wherein said selected parameter is cardiac output, $CO_i$, and wherein said predetermined expression is:

$$CO_i \;=\; 60\, \frac{P_{mmi}}{R_{pi}} \qquad \text{mL/min}$$

wherein

$P_{mmi}$ =     pseudo mean arterial pressure.

PLETHYSMO-
GRAPH
AMPLIFIER

PHOTO-
PLETHYMO-
GRAPH

MICRO-
COMPUTER

A/D
CONVERTER

DISPLAY

| S | A₁ | A₂ | M | D |

FIG.1

VOLTS

O

TIME

FIG.2

0160994

Fig.3

$$Pdi = Psie - \frac{\Delta tdi}{Xi}$$

$$Gi = \frac{\Delta Viav}{\Delta Vi} = \frac{PPiav}{PPi}$$

Fig.5

Fig. 4

0160994

START

SAMPLE INPUT

COMPLETE
CYCLE i ── NO

YES

FIND    Vimax
         Vimin
         Vimax
     $\triangle$Vi
     $\triangle$Vivm
     $\triangle$tivm
       Ti
   $\triangle$ti$_\phi$
       Ti
   $\triangle$t'i$_\phi$
   $\triangle$t"i$_\phi$

COMPUTE    Ppi
           Pmi
           Psi
           Pdi
           Rpi
           COi
           SVi
           HRi

DISPLAY    Ps
           Pd
           CO
           SV
           HR

STOP

FIG. 6